# EUROPEAN PATENT APPLICATION

(11) **EP 4 115 902 A1**
(43) Date of publication of application: **11.01.2023**
(21) Application number: 21763694.3
(22) Date of filing: 05.03.2021
(51) Int. Cl.: A61K 39/395, A61P 35/00, C07K 16/28, A61K 38/21, A61K 31/5025

(54) **COMBINED USE OF CTB006 AND PONATINIB**

(30) Priority: 06.03.2020 WO PCT/CN2020/078158
(71) Applicant: Beijing Sinotau Bio-Pharmaceuticals Technology Co., Ltd., Beijing 102206 (CN)
(72) Inventor: ZHONG, Xiaoyan, Beijing 102206 (CN); CAO, Fengqi, Beijing 102206 (CN); LI, Zhe, Beijing 102206 (CN)
(74) Representative: Longland, Emma Louise
(86) International application number: PCT/CN2021/079403
(87) International publication number: WO 2021/175326

(57) **Abstract**

Provided herein are a combination of antitumor medicaments comprising a TRATL-R2 agonistic antibody (e.g., CTB006) and Ponatinib or its hydrochloride. Also provided herein are a method of treating tumors that include administering therapeutically effective amounts of a TRATL-R2 agonistic antibody and Ponatinib or its hydrochloride to a patient. The synergistic effect of these two medicaments helps to enhance the effect of tumor treatment.

## Description

### FIELD OF THE INVENTION

The invention relates to a combination of antitumor medicaments, and in particular to a combination of antitumor medicaments comprising a TRAIL-R2 agonistic antibody (e.g., CTB006 antibody) and Ponatinib. The invention also relates to a method of treating tumors that combine a TRAIL-R2 agonistic antibody and Ponatinib.

### BACKGROUND OF THE INVENTION

Tumor necrosis factor-related apoptosis-inducing ligand (TRAIL), also known in literatures as Apo2L or TNFSF10, was identified in the 1990s and belongs to the tumor necrosis factor (TNF) superfamily. TRAIL is expressed in immune system cells such as NK cells, T cells, macrophages and dendritic cells. Mature TRAIL exists in either a membrane-bound form or a soluble form, where the soluble form is the extracellular active fraction obtained by hydrolysis of membrane-bound TRAIL by proteases. Both forms of TRAIL can form trimers and can induce apoptosis of target cells by interacting with the TRAIL receptor (TRAIL-R) on the target cells. Five receptors with TRAIL binding activity have been identified in humans. Two of them, TRAIL-R1 (or DR4) and TRAIL-R2 (or DR5), transduce apoptotic signals, whereas the other three, TRAIL-R3 (DcR1), TRAIL-R4 (DcR2) and osteoprotegerin (OPG), do not transduce apoptotic signals. All five receptors of TRAIL have significant homology in their extracellular ligand-binding domains, and the intracellular fragments of TRAIL-R1 and TRAIL-R2 contain a conserved functional domain, the so-called "death domain", which is responsible for transducing apoptotic signals. The direct use of recombinant human TRAIL to induce apoptosis in human tumor cells has been found to have a short biological half-life and poor stability. Some research institutions and biologic companies have developed different TRAIL-R agonistic monoclonal antibodies in an attempt to replace TRAIL to enhance the antitumor effect, but the clinical effectiveness remains limited.

Ponatinib hydrochloride is a multi-targeted kinase inhibitor manufactured and marketed by ARIAD Pharmaceuticals under the trade name Iclusig^{®}. Its clinical indication is limited to hematologic neoplasms, such as certain types of chronic myeloid leukemia (CML) and acute lymphoblastic leukemia (ALL).

### SUMMARY OF THE INVENTION

In one aspect, the invention provides a combination of antitumor medicaments comprising an apoptosis inducer and a multi-targeted kinase inhibitor.

In some embodiments, the apoptosis inducer is a TRAIL-R2 binding agent.

In some embodiments, the TRAIL-R2 binding agent is a TRAIL-R2 agonist.

In some embodiments, the TRAIL-R2 agonist is a TRAIL-R2 natural ligand TRAIL or a TRAIL-R2 agonistic antibody.

In some embodiments, the TRAIL-R2 agonistic antibody is B273, Conatumumab, CTB006 antibody, Hexabody-DR5/DR5 or CTB003 antibody.

In some embodiments, the multi-targeted kinase inhibitor is Ponatinib or a pharmaceutically acceptable salt thereof.

In some embodiments, the multi-targeted kinase inhibitor is Ponatinib hydrochloride.

In some embodiments, the tumor expresses TRAIL-R2 on the cell surface.

In some embodiments, the tumor is breast cancer, pancreatic cancer, colorectal cancer, gastric cancer, lung cancer, liver cancer, esophageal cancer or ovarian cancer.

In some embodiments, the apoptosis inducer and the multi-targeted kinase inhibitor are present in the same or different pharmaceutical composition or pharmaceutical kit.

In some embodiments, the apoptosis inducer is in a dosage form suitable for intravenous administration and the multi-targeted kinase inhibitor is in a dosage form suitable for oral administration.

In another aspect, the invention provides a method of treating a tumor in a subject comprising administering to the subject a therapeutically effective amount of an apoptosis inducer and a multi-targeted kinase inhibitor simultaneously, sequentially or separately.

In some embodiments, the apoptosis inducer is a TRAIL-R2 binding agent.

In some embodiments, the TRAIL-R2 binding agent is a TRAIL-R2 agonist.

In some embodiments, the TRAIL-R2 agonist is a TRAIL-R2 natural ligand TRAIL or a TRAIL-R2 agonistic antibody.

In some embodiments, the TRAIL-R2 agonistic antibody is B273, Conatumumab, CTB006 antibody, Hexabody-DR5/DR5 or CTB003 antibody.

In some embodiments, the multi-targeted kinase inhibitor is Ponatinib or a pharmaceutically acceptable salt thereof.

In some embodiments, the multi-targeted kinase inhibitor is Ponatinib hydrochloride.

In some embodiments, the tumor expresses TRAIL-R2 on the cell surface.

In some embodiments, the tumor is breast cancer, pancreatic cancer, colorectal cancer, gastric cancer, lung cancer, liver cancer, esophageal cancer or ovarian cancer.

In some embodiments, the apoptosis inducer is administered by intravenous injection and the multi-targeted kinase inhibitor is administered orally.

In another aspect, the invention provides use of an apoptosis inducer in the preparation of a medicament for co-administration in combination with a multi-targeted kinase inhibitor to treat a tumor.

In some embodiments, the apoptosis inducer is a TRAIL-R2 binding agent.

In some embodiments, the TRAIL-R2 binding agent is a TRAIL-R2 agonist.

In some embodiments, the TRAIL-R2 agonist is a TRAIL-R2 natural ligand TRAIL or a TRAIL-R2 agonistic antibody.

In some embodiments, the TRAIL-R2 agonistic antibody is B273, Conatumumab, CTB006 antibody, Hexabody-DR5/DR5 or CTB003 antibody.

In some embodiments, the multi-targeted kinase inhibitor inhibits activation of the MEK/ERK signaling pathway.

In some embodiments, the multi-targeted kinase inhibitor is Ponatinib or a pharmaceutically acceptable salt thereof.

In some embodiments, the multi-targeted kinase inhibitor is Ponatinib hydrochloride.

In some embodiments, the tumor expresses TRAIL-R2 on the cell surface.

In some embodiments, the tumor is breast cancer, pancreatic cancer, colorectal cancer, gastric cancer, lung cancer, liver cancer, esophageal cancer or ovarian cancer.

In another aspect, the invention provides a method of inducing apoptosis in a tumor cell comprising contacting said tumor cell with an apoptosis inducer and a multi-targeted kinase inhibitor.

In some embodiments, the apoptosis inducer is CTB006 antibody, the multi-targeted kinase inhibitor is Ponatinib or hydrochloride thereof.

In another aspect, the invention provides a method of enhancing antitumor effects in a subject intolerant to Ponatinib or a pharmaceutically acceptable salt thereof, comprising administering CTB006 antibody to the subject.

### DESCRIPTION OF THE DRAWINGS

Figure 1 shows tumor killing effect of CTB006 in combination with Ponatinib on MCF-7 cells.
Figure 2 shows tumor killing effect of CTB006 in combination with Ponatinib on S2Lm7AA cells.
Figure 3 shows tumor killing effect of CTB006 in combination with Ponatinib on S2VP10 cells.
Figure 4 shows tumor killing effect of CTB006 in combination with Ponatinib on HT29 cells.
Figure 5 shows tumor killing effect of CTB006 in combination with Ponatinib on SGC-7901 cells.
Figure 6 shows tumor killing effect of CTB006 in combination with Ponatinib on N87 cells.
Figure 7 shows tumor killing effect of CTB006 in combination with Ponatinib on A549 cells.
Figure 8 shows tumor killing effect of CTB006 in combination with Ponatinib on SK-HEP-1 cells.
Figure 9 shows tumor killing effect of CTB006 in combination with Ponatinib on Huh-1 cells.
Figure 10 shows tumor killing effect of CTB006 in combination with Ponatinib on TE1 cells.
Figure 11 shows the expression of DR5 in primary tumor cells from ovarian cancer ascites.
Figure 12 shows killing effect of CTB006 antibody and Ponatinib on primary tumor cells S1 (A) and S2 (B).
Figure 13 shows the efficacy of CTB006 in combination with Ponatinib on MIA-Paca-2, a transplanted tumor model of pancreatic cancer. (A) Change in tumor volume, (B) Survival curve.
Figure 14 shows the effect of CTB006 in combination with Ponatinib on the PDX transplantation tumor model CS237.
Figure 15 shows the effect of CTB006 in combination with Ponatinib on the PDX transplantation tumor model CS263. (A) change in tumor volume, (B) survival curve.
Figure 16 shows the effect of CTB006 in combination with Ponatinib on the PDX transplantation tumor model CS264.
Figure 17 shows the effect of CTB006 in combination with Ponatinib on the PDX transplantation tumor model CS225.
Figure 18 shows the effect of CTB006 in combination with Ponatinib on the PDX transplantation tumor model CS266.
Figure 19 shows the effect of CTB006 in combination with Ponatinib on PDX transplantation tumor model GAS033.
Figure 20 shows killing effect of different types of DR5 agonists in combination with Ponatinib on A549 cell.
Figure 21 shows killing effect of different types of DR5 agonists in combination with Ponatinib on S2VP10 cell.
Figure 22 shows killing effect of different types of DR5 agonists in combination with Ponatinib on MCF7 cell.
Figure 23 shows activation of apoptosis and proliferation signalling pathway in different tumor cells with different drug treatment (colon cancer cells SW1116 (A), gastric cancer cells 7901 (B), colorectal cancer HT29 (C), pancreatic cancer S2Lm7AA (D)). lane-1: cell control; lane-2: 1000ng/ml CTB006 treatment group; Lane-3: 1µM Ponatinib treatment group; Lane-4: the combination group (1000ng/ml CTB006 and 1µM Ponatinib).

### DETAILED DESCRIPTION OF THE INVENTION

Unless otherwise stated, all technical and scientific terms used herein have the meaning commonly understood by those of ordinary skill in the art.

The term "apoptosis inducer" refers to various chemical agents (e.g., small molecule drugs), biological agents (e.g., polypeptides, antibodies), or even physical treatments (e.g., radiation) that promote apoptosis of cells (e.g., tumor cells) in vitro or in vivo.

The term "agonistic antibody" refers to an antibody that binds to and triggers the biological activity of a corresponding antigen. In some embodiments, the antigen is TRAIL-R2, and the agonistic antibody triggers the apoptotic activity of its intracellular death domain when bound to it.

The term "kinase inhibitor" refers to a chemical or biological agent that can inhibit, attenuate, or eliminate the phosphorylation of a substrate protein by a kinase. Accordingly, the term "multi-targeted kinase inhibitor" refers to a kinase inhibitor that can act on multiple kinases. For example, when administered in vivo, a multi-targeted kinase inhibitor can inhibit multiple signaling pathways. Examples of known multi-targeted kinase inhibitor include Lapatinib (targeting EGFR and HER2), Bosutilib (targeting SRC and ABL), Imatinib (targeting BCR-ABL, C-KIT and PDGFR), Ponatinib (targeting ABL, PDGFR, VEGFR, Src, etc.), etc. In some embodiments, the multi-targeted kinase inhibitor is Ponatinib or a pharmaceutically acceptable salt thereof.

The term "CTB006" or "CTB006 antibody" refers to an agonistic antibody targeting TRAIL-R2. The mouse-mouse hybridoma cells producing the CTB006 antibody have been deposited at the Chinese General Microbiological Culture Collection Center (CGMCC) on April 11, 2006 under Deposit No. 1691, see the publication of PCT application WO2016/074245 (incorporated herein by reference in its entirety). The antibody has the heavy chain CDR amino acid sequences of SYFIH (SEQ ID NO: 1), WIYPGNVNTKYSEKFKG (SEQ ID NO: 2) and GEAGYFD (SEQ ID NO: 3), and the light chain CDR amino acid sequences of KASQDVSTAVA (SEQ ID NO: 4), WASTRHT (SEQ ID NO: 5) and QQHYRTPW (SEQ ID NO: 6). The PCT application also describes the light and heavy chain amino acid sequences of the antibody as well as the humanized chimeric antibody sequence. It should be understood by those skilled in the art that where the sequence of antibody CDR is known, substitutions of amino acids in other portions of the sequence of the antibody or even a few amino acids in that CDR can be made without altering or without substantially altering its binding activity. Furthermore, it is foreseeable that other antibodies with the same epitope specificity as this antibody should also have corresponding pro-apoptotic activity. Thus, it is possible to consider replacing CTB006 antibody described herein with these antibody variants for use in combination with said kinase mechanism. These antibody variants are also included in the scope of the present invention. When CTB006 is mentioned in the following examples, a humanized chimeric antibody thereof is used.

The term "synergism" or "synergistic effect" refers to that the effect between two or more agents, entities, factors or substances that produces is greater than the sum of their respective effects. In some embodiments, for example, the synergistic effect between CTB006 and Ponatinib is determined by coefficient of drug interaction (i.e., CDI). In some embodiments, CDI is calculated according to the following formula: CDI =AB/AxB, where AB is the ratio of the detection values of the two-drug combination group to the control group (e.g., survival rate of the drug combination group), and A or B is the ratio of the detection values of each drug alone to the control group (e.g., survival rate of the drug alone group). If CDI < 1, the nature of action of the two drugs can be considered synergistic, and if CDI < 0.7, the synergism of the two drugs can be considered very significant; if CDI = 1, the nature of action of the two drugs is considered additive; if CDI > 1, the nature of action of the two drugs is considered antagonistic.

The term "subject" refers to an animal (preferably a human) that has or is suspected of having a disease, or, in the case of predicting susceptibility, "subject" may also include a healthy individual. The term is often used interchangeably with the term "patient", "sick person", "detection object", "treatment object", etc.

The term "pharmaceutically acceptable salt" refers to a relatively non-toxic inorganic or organic acid addition salt of a drug. The nature of the salt is usually not critical, provided that it is pharmaceutically acceptable. "Pharmaceutically acceptable salt", as used herein, refers to an inorganic or organic acid addition salt that is essentially harmless to an animal or a human, such as hydrochloride, hydrobromide, phosphate, sulfate, formate, acetate, ascorbate, benzenesulfonate, benzoate, cinnamate, citrate, fumarate, glutamate, hydroxyacetate, lactate, maleate, methanesulfonate, salicylate, stearate, succinate, tartarate, etc. Such salts can be formed by methods known to those skilled in the art.

In some embodiments, CTB006 antibody is administered in combination with Ponatinib. In other embodiments, CTB006 antibody is administered in combination with a pharmaceutically acceptable salt (e.g., hydrochloride) of Ponatinib.

The term "therapeutically effective amount" refers to an amount of an active compound or pharmaceutical composition sufficient to elicit a biological or medical response in a subject as desired by the clinician. A "therapeutically effective amount" may generally be determined by those skilled in the art based on the route of administration, the weight, the age, the condition of the subject, and other factors. For example, a typical daily dose may range from 0.01 mg to 100 mg of active ingredient per kg of body weight.

In some aspects of the present invention, a combination of medicaments comprising an apoptosis inducer and a multi-targeted kinase inhibitor is provided. As used herein, the term "a combination of medicaments" means not only that the apoptosis inducer and the multi-targeted kinase inhibitor may be present together in the same pharmaceutical formulation, but also that the apoptosis inducer and the multi-targeted kinase inhibitor may be present in the same pharmaceutical kit as separate pharmaceutical formulations, or even that the apoptosis inducer and the multi-targeted kinase inhibitor may be present as separate pharmaceutical formulations in separate pharmaceutical kits. In fact, such a "combination of medicaments" is used whenever the apoptosis inducer and the multi-targeted kinase inhibitor are administered in such a way that they are both present in the subject at a given time.

An apoptosis inducer and/or a multi-targeted kinase inhibitor are often formulated with pharmaceutically acceptable carriers to form pharmaceutical formulations or pharmaceutical compositions. As used herein, "pharmaceutically acceptable carrier" refers to a solid or liquid diluent, filler, antioxidant, stabilizer, etc. that is safe for administration to an animal or a human. These substances are suitable for administration to a human and/or an animal without undue adverse side effect and for maintaining the viability of the drug or active agent therein. Depending on the route of administration, a variety of different carriers well known in the art may be used, including, but not limited to, sugars, starch, cellulose and derivatives thereof, maltose, gelatin, talc, calcium sulfate, vegetable oils (e.g., castor oil), synthetic oils, polyols, alginic acid, phosphate buffers, emulsifiers, isotonic saline, and/or pyrogen-free water, etc. Suitable routes of administration for use include, for example, oral, intravenous infusion, intramuscular injection, subcutaneous injection, subperitoneal, rectal, sublingual, or inhalation, transdermal, and other routes. Accordingly, an apoptosis inducer and/or a multi-target kinase inhibitor can be formulated together with these pharmaceutically acceptable carriers into any clinically acceptable dosage form, such as tablet, granule, powder, capsule, injectable formulation, suppositorie, drop, external plaster, ointment, medicated oil, or spray, and the like.

In some aspects of the present invention, a method of treating a tumor (or cancer) by co-administration of an apoptosis inducer and a multi-targeted kinase inhibitor. The term "co-administration" as used herein, for example, for a combination of CTB006 antibody and Ponatinib, includes sequentially administering CTB006 antibody and Ponatinib separately, such as administering CTB006 antibody before or after Ponatinib administration, and further includes simultaneous administration of Ponatinib and CTB006 antibody in the same pharmaceutical formulation or in separate pharmaceutical formulations. In embodiments of sequential administration, typically Ponatinib and the CTB006 antibody are present together in the subject for at least part of the time. In some embodiments of co-administration, Ponatinib and the CTB006 antibody may have a synergistic effect, such as where the amount of one of the drugs is lower than the therapeutically effective amount thereof when administered alone, or preferably, where the amount of both drugs is lower than the therapeutically effective amount thereof when administered alone.

Some embodiments of the present invention are based, at least in part, on the inventors' finding that co-administration of an apoptosis inducer and a multi-targeted kinase inhibitor produces significant synergistic effect. In addition to the synergistic effect demonstrated by CDI as described above, the synergistic effect can be seen in 1) generation of novel therapeutic effect, such as the ability to treat tumor that do not respond to treatment with either the apoptosis inducer or the multi-targeted kinase inhibitor, 2) reduction of therapeutic side effect, 3) extension of the dosing interval, 4) reduction of the duration of treatment, and so on.

The combination of antitumor medicaments and method of treating tumor provided herein utilize the synergistic effect of an apoptosis inducer and a multi-target kinase inhibitor to enhance tumor treatment. In some embodiments, the use of an apoptosis inducer (e.g., CTB006 antibody) enhances the tumor growth inhibitory effect of a kinase inhibitor (e.g., Ponatinib). In some embodiments, the use of an apoptosis inducer (e.g., CTB006 antibody) reduces the duration of treatment with a kinase inhibitor (e.g., Ponatinib), thereby improving patient tolerability.

The present invention is further described by specific examples below.

### Example 1 CTB006 in combination with Ponatinib significantly enhances apoptosis of tumor cells

The effect of the combination of the two drugs was validated using tumor cell lines and primary tumor cells derived from cancer patient, respectively.

### 10.1 The combination of CTB006 and Ponatinib synergistically kills multiple tumor cell lines.

The in vitro pharmacodynamics of CTB006 in combination with Ponatinib was verified by in vitro combined killing effect on tumor cell lines from multiple cancers.

The interaction between CTB006 antibody and Ponatinib: we used coefficient of drug interaction (CDI)^{[1,2]} to evaluate the combined effect of CTB006 antibody and chemotherapeutic drugs. CDI was calculated according to the following formula: CDI =AB/AxB; AB was the ratio of the detection values of the two-drug combination group to the control group (survival rate of the drug combination group), and A or B was the ratio of the detection values of each individual drug to the control group (survival rate of the drug alone group). If CDI<1, the nature of action of the two drugs was proved to be synergistic; if CDI<0.7, the synergistic effect of the two drugs was very significant; if CDI=1, the nature of action of the two drugs was additive; if CDI>1, the nature of action of the two drugs was antagonistic.

The experiment was performed as follows: in a 96-well plate, 3000 tumor cells in logarithmic growth phase were added to each well along with different concentrations of CTB006 antibody and Ponatinib (hydrochloride form, CAS: 1114544-31-8) (Pharmadodis). The survival of each tumor cell was observed by ATPlite method after incubation at 37°C,5% CO₂ for 24h.

### 10.1.1 CTB006 in combination with Ponatinib could synergistically kill breast cancer cells MCF-7.

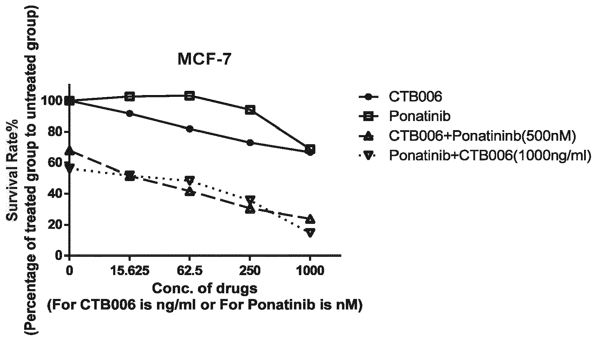

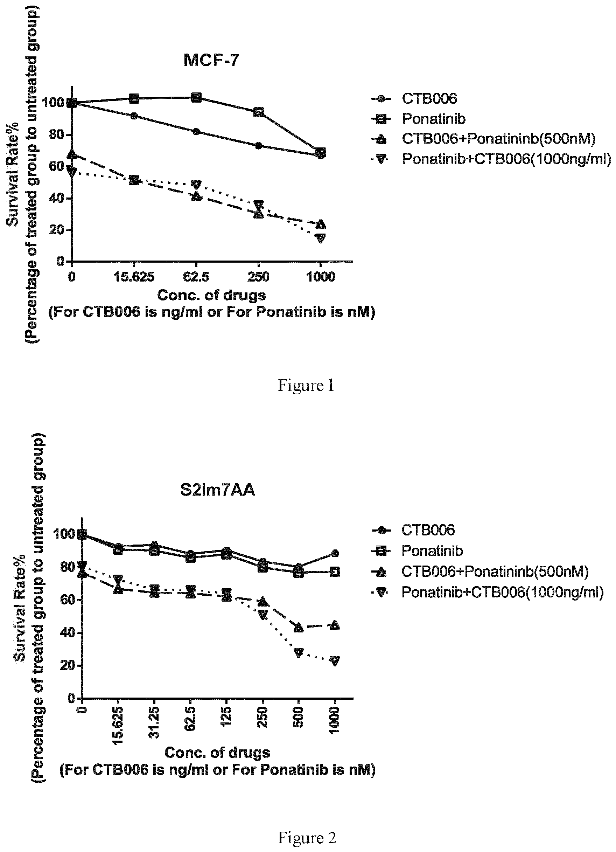

The results were shown in Figure 1. CTB006 and Ponatinib alone had some degree of killing effect on MCF-7. The killing effect was more obvious when the two were used in combination. The CDI values were less than 1 for co-administration of different concentrations of CTB006 antibody and 500 nM of Ponatinib, and less than 1 for co-administration of different concentrations of Ponatinib and 1000 ng/ml of CTB006 (see Table 1), indicating that the effect of co-administration of CTB006 antibody and Ponatinib on breast cancer MCF-7 cells was synergistic (see Figure 1).

**Table 1 :CTB006 and Ponatinib synergistically killed MCF-7 indicated by CDI**

| CTB006 alone | | Ponatinib alone | | Varying concentrations of CTB006+500nM Ponatinib | | | Varying concentrations of Ponatinib+1000ng/ml CTB006 | | |
|---|---|---|---|---|---|---|---|---|---|
| Con. (ng/ml) | Survival Rate (%) | Con. (nM) | Survival Rate (%) | Con. (ng/ml) | Survival Rate (%) | **CDT** | Con. (nM) | Survival Rate (%) | **CDT** |
| 0 | 100.00% | 0 | 100.00% | 0 | 67.82% | | 0 | 56.17% | |
| 15.625 | 91.75% | 15.625 | 102.70% | 15.625 | 51.48% | **0.83*** | 15.625 | 51.78% | **0.90*** |
| 62.5 | 81.78% | 62.5 | 103.26% | 62.5 | 41.61% | **0.75*** | 62.5 | 48.36% | **0.83*** |
| 250 | 72.88% | 250 | 94.14% | 250 | 30.48% | **0.62**** | 250 | 35.57% | **0.67**** |
| 1000 | 66.69% | 1000 | 68.81% | 1000 | 23.82% | **0.53**** | 1000 | 14.64% | **0.38**** |

| | | | | | | | | | |
|---|---|---|---|---|---|---|---|---|---|
| *synergistic effect (CDI<1); **significant synergistical effect (CDI<0.7) | | | | | | | | | |

### 10.1.2 CTB006 in combination with Ponatinib could synergistically kill pancreatic cancer cells S2Lm7AA.

In the experiments of pancreatic cancer cells S2Lm7AA, CTB006 and Ponatinib alone had no killing effect on S2Lm7AA. The combination of them had a killing effect. The CDI values were less than 1 for co-administration of different concentrations of CTB006 antibody and 500 nM of Ponatinib, and less than 1 for co-administration of different concentrations of Ponatinib and 1000 ng/ml of CTB006 (see Table 2), indicating that the effect of co-administration of CTB006 antibody and Ponatinib on pancreatic cancer S2Lm7AA cells was synergistic (see Figure 2).

**Table 2: Survival rate and CDI of two-drug effect of CTB006 antibody and Ponatinib on S2Lm7AA cells**

| CTB006 alone | | Ponatinib alone | | Varying concentrations of CTB006+500nM Ponatinib | | | Varying concentrations of Ponatinib+1000ng/ml CTB006 | | |
|---|---|---|---|---|---|---|---|---|---|
| Con. (ng/ml) | Survival Rate (%) | Con. (nM) | Survival Rate (%) | Con. (ng/ml) | Survival Rate (%) | **CDT** | Con. (nM) | Survival Rate (%) | **CDT** |
| 0 | 100.00% | 0 | 100.00% | 0 | 76.76% | | 0 | 80.72% | |
| 15.625 | 92.74% | 15.625 | 90.79% | 15.625 | 66.75% | **0.94*** | 15.625 | 72.38% | **0.99*** |
| 31.25 | 93.57% | 31.25 | 90.05% | 31.25 | 64.55% | **0.90*** | 31.25 | 66.33% | **0.91*** |
| 62.5 | 88.20% | 62.5 | 85.82% | 62.5 | 64.15% | **0.95*** | 62.5 | 66.03% | **0.95*** |
| 125 | 90.25% | 125 | 87.89% | 125 | 62.23% | **0.90*** | 125 | 64.03% | **0.90*** |
| 250 | 83.40% | 250 | 79.85% | 250 | 59.15% | **0.92*** | 250 | 50.94% | **0.79*** |
| 500 | 80.24% | 500 | 76.76% | 500 | 43.39% | **0.70**** | 500 | 27.70% | **0.45**** |
| 1000 | 88.45% | 1000 | 77.11% | 1000 | 44.98% | **0.66**** | 1000 | 22.73% | **0.37**** |

| | | | | | | | | | |
|---|---|---|---|---|---|---|---|---|---|
| *synergistic effect (CDI<1); **significant synergistical effect (CDI<0.7) | | | | | | | | | |

### 10.1.3 CTB006 in combination with Ponatinib could synergistically kill pancreatic cancer cells S2VP10.

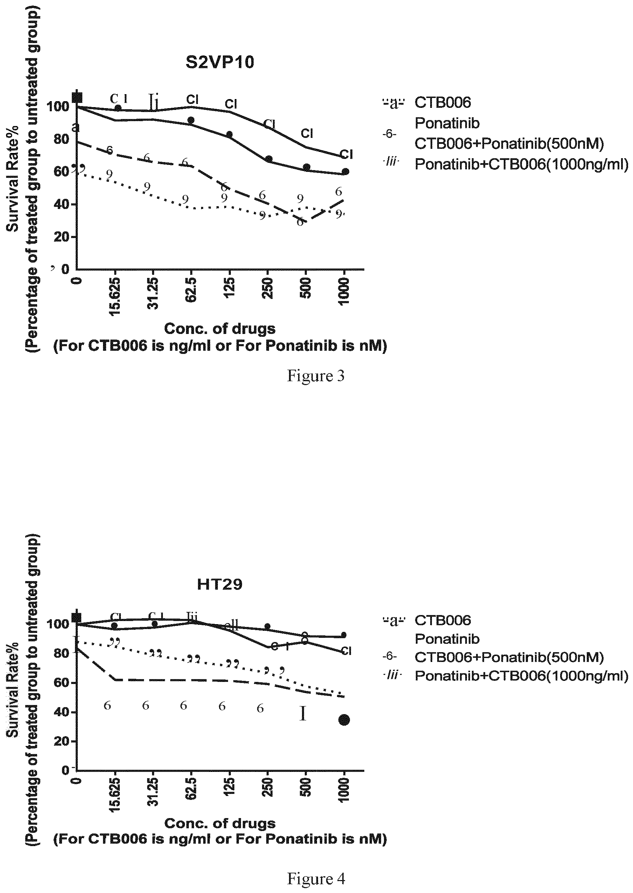

In the experiments of the pancreatic cancer cell S2VP10, CTB006 and Ponatinib alone had no killing effect on S2VP10. The combination of them had a killing effect. The CDI values were less than 1 for co-administration of different concentrations of CTB006 antibody and 500 nM of Ponatinib, and less than 1 for co-administration of different concentrations of Ponatinib and 1000 ng/ml of CTB006 (see Table 3), indicating that the effect of co-administration of CTB006 antibody and Ponatinib on pancreatic cancer S2VP10 cells was synergistic (see Figure 3).

**Table 3: Survival rate and CDI of two-drug effect of CTB006 antibody and Ponatinib on S2VP10 cells**

| CTB006 alone | | Ponatinib alone | | Varying concentrations of CTB006+500nM Ponatinib | | | Varying concentrations of Ponatinib+1 000ng/ml CTB006 | | |
|---|---|---|---|---|---|---|---|---|---|
| Con. (ng/ml) | Survival Rate (%) | Con. (nM) | Survival Rate (%) | Con. (ng/ml) | Survival Rate (%) | **CDT** | Con. (nM) | Survival Rate (%) | **CDT** |
| 0 | 100.00% | 0 | 100.00% | 0 | 78.49% | | 0 | 59.07% | |
| 15.625 | 91.59% | 15.625 | 98.02% | 15.625 | 70.55% | **0.98*** | 15.625 | 53.65% | **0.93*** |
| 31.25 | 92.16% | 31.25 | 97.47% | 31.25 | 66.02% | **0.91*** | 31.25 | 45.13% | **0.78*** |
| 62.5 | 88.98% | 62.5 | 99.87% | 62.5 | 63.55% | **0.91*** | 62.5 | 37.52% | **0.64**** |
| 125 | 81.17% | 125 | 96.84% | 125 | 49.47% | **0.78*** | 125 | 38.57% | **0.67**** |
| 250 | 66.48% | 250 | 87.54% | 250 | 40.56% | **0.78*** | 250 | 32.73% | **0.63**** |
| 500 | 60.87% | 500 | 75.12% | 500 | 29.43% | **0.62**** | 500 | 38.16% | **0.86*** |
| 1000 | 58.50% | 1000 | 69.06% | 1000 | 42.90% | **0.93*** | 1000 | 34.19% | **0.84*** |

| | | | | | | | | | |
|---|---|---|---|---|---|---|---|---|---|
| *synergistic effect (CDI<1); **significant synergistical effect (CDI<0.7) | | | | | | | | | |

### 10.1.4 CTB006 in combination with Ponatinib could synergistically kill colorectal cancer cells HT29.

CDI values were less than 1 for co-administration of different concentrations of CTB006 antibody and 500 nM of Ponatinib, and less than 1 for co-administration of different concentrations of Ponatinib and 1000 ng/ml of CTB006 (see Table 4), indicating that the effect of co-administration of CTB006 antibody and Ponatinib on colorectal cancer HT29 cells was synergistic (see Figure 4).

**Table 4: Survival rate and CDI of two-drug effect of CTB006 antibody and Ponatinib on HT29 cells**

| CTB006 alone | | Ponatinib alone | | Varying concentrations of CTB006+500nM Ponatinib | | | Varying concentrations of Ponatinib+1 000ng/ml CTB006 | | |
|---|---|---|---|---|---|---|---|---|---|
| Con. (ng/ml) | Survival Rate (%) | Con. (nM) | Survival Rate (%) | Con. (ng/ml) | Survival Rate (%) | **CDT** | Con. (nM) | Survival Rate (%) | **CDT** |
| 0 | 100.00% | 0 | 100.00% | 0 | 83.63% | | 0 | 88.05% | |
| 15.625 | 96.45% | 15.625 | 102.78% | 15.625 | 62.11% | **0.77*** | 15.625 | 84.71% | **0.94*** |
| 31.25 | 97.76% | 31.25 | 103.51% | 31.25 | 61.95% | **0.76*** | 31.25 | 78.99% | **0.87*** |
| 62.5 | 100.99% | 62.5 | 102.93% | 62.5 | 62.05% | **0.73*** | 62.5 | 74.78% | 0.83* |
| 125 | 98.48% | 125 | 95.77% | 125 | 61.47% | **0.75*** | 125 | 71.45% | **0.85*** |
| 250 | 96.21 % | 250 | 84.39% | 250 | 59.33% | **0.74*** | 250 | 66.74% | **0.90*** |
| 500 | 91.97% | 500 | 87.91% | 500 | 53.87% | **0.70**** | 500 | 57.63% | **0.74*** |
| 1000 | 91.35% | 1000 | 80.77% | 1000 | 50.67% | **0.66**** | 1000 | 52.59% | **0.74*** |

| | | | | | | | | | |
|---|---|---|---|---|---|---|---|---|---|
| ^{∗}synergistic effect (CDI<1); ^{∗∗}significant synergistical effect (CDI<0.7) | | | | | | | | | |

### 1.1.5 CTB006 in combination with Ponatinib could synergistically kill gastric cancer cells SGC-7901.

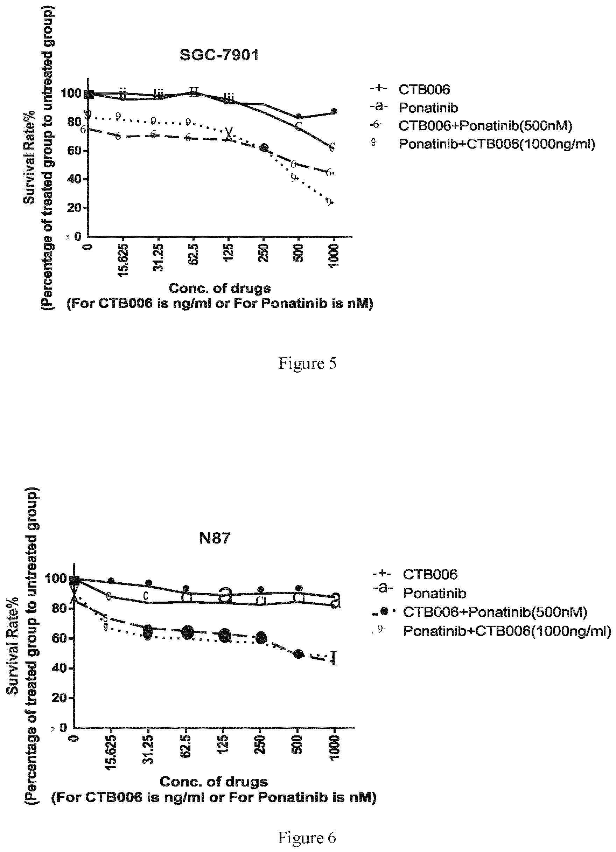

CDI values were less than 1 for co-administration of different concentrations of CTB006 antibody and 500 nM of Ponatinib, and less than 1 for co-administration of different concentrations of Ponatinib and 1000 ng/ml of CTB006 (see Table 5), indicating that the effect of co-administration of CTB006 antibody and Ponatinib on gastric cancer SGC- 7901 cells was synergistic (see Figure 5).

**Table 5: Survival rate and CDI of two-drug effect of CTB006 antibody and Ponatinib on SGC-7901 cells**

| CTB006 alone | | Ponatinib alone | | Varying concentrations of CTB006+500nM Ponatinib | | | Varying concentrations of Ponatinib+1 000ng/ml CTB006 | | |
|---|---|---|---|---|---|---|---|---|---|
| Con. (ng/ml) | Survival Rate (%) | Con. (nM) | Survival Rate (%) | Con. (ng/ml) | Survival Rate (%) | **CDT** | Con. (nM) | Survival Rate (%) | **CDT** |
| 0 | 100.00% | 0 | 100.00% | 0 | 75.13% | | 0 | 83.13% | |
| 15.625 | 95.71% | 15.625 | 100.08% | 15.625 | 69.93% | **0.97*** | 15.625 | 81.51% | **0.98*** |
| 31.25 | 96.18% | 31.25 | 98.30% | 31.25 | 70.64% | **0.98*** | 31.25 | 79.31% | **0.97*** |
| 62.5 | 101.02% | 62.5 | 99.90% | 62.5 | 68.47% | **0.90*** | 62.5 | 78.79% | **0.95*** |
| 125 | 93.24% | 125 | 95.83% | 125 | 67.66% | **0.97*** | 125 | 72.01% | **0.90*** |
| 250 | 92.38% | 250 | 86.80% | 250 | 60.89% | **0.88*** | 250 | 61.00% | **0.85*** |
| 500 | 82.75% | 500 | 75.77% | 500 | 50.17% | **0.81*** | 500 | 39.65% | **0.63**** |
| 1000 | 86.19% | 1000 | 61.42% | 1000 | 44.05% | **0.68**** | 1000 | 22.79% | **0.45**** |

| | | | | | | | | | |
|---|---|---|---|---|---|---|---|---|---|
| *synergistic effect (CDI<1); **significant synergistical effect (CDI<0.7) | | | | | | | | | |

### 1.1.6 CTB006 in combination with Ponatinib could synergistically kill gastric cancer cells N87.

CDI values were less than 1 for co-administration of different concentrations of CTB006 antibody and 500 nM of Ponatinib, and less than 1 for co-administration of different concentrations of Ponatinib and 1000 ng/ml of CTB006 (see Table 6), indicating that the effect of co-administration of CTB006 antibody and Ponatinib on gastric cancer N87 cells was synergistic (see Figure 6).

**Table 6: Survival rate and CDI of two-drug effect of CTB006 antibody and Ponatinib on N87 cells**

| CTB006 alone | | Ponatinib alone | | Varying concentrations of CTB006+500nM Ponatinib | | | Varying concentrations of Ponatinib+1000ng/ml CTB006 | | |
|---|---|---|---|---|---|---|---|---|---|
| Con. (ng/ml) | Survival Rate (%) | Con. (nM) | Survival Rate (%) | Con. (ng/ml) | Survival Rate (%) | **CDT** | Con. (nM) | Survival Rate (%) | **CDT** |
| 0 | 100.00% | 0 | 100.00% | 0 | 85.31% | | 0 | 90.82% | |
| 15.625 | 97.57% | 15.625 | 87.89% | 15.625 | 72.91% | **0.88*** | 15.625 | 66.45% | **0.83*** |
| 31.25 | 94.92% | 31.25 | 83.79% | 31.25 | 66.90% | **0.83*** | 31.25 | 60.92% | **0.80*** |
| 62.5 | 90.33% | 62.5 | 84.36% | 62.5 | 64.91% | **0.84*** | 62.5 | 60.08% | **0.78*** |
| 125 | 89.03% | 125 | 83.71% | 125 | 63.05% | **0.83*** | 125 | 58.30% | **0.77*** |
| 250 | 90.20% | 250 | 82.65% | 250 | 60.65% | **0.79*** | 250 | 57.29% | **0.76*** |
| 500 | 90.69% | 500 | 84.46% | 500 | 49.32% | **0.64**** | 500 | 50.14% | **0.65**** |
| 1000 | 87.70% | 1000 | 82.19% | 1000 | 44.53% | **0.60**** | 1000 | 47.85% | **0.64**** |

| | | | | | | | | | |
|---|---|---|---|---|---|---|---|---|---|
| *synergistic effect (CDI<1); **significant synergistical effect (CDI<0.7) | | | | | | | | | |

### 1.1.7 CTB006 in combination with Ponatinib could synergistically kill lung cancer cells A549.

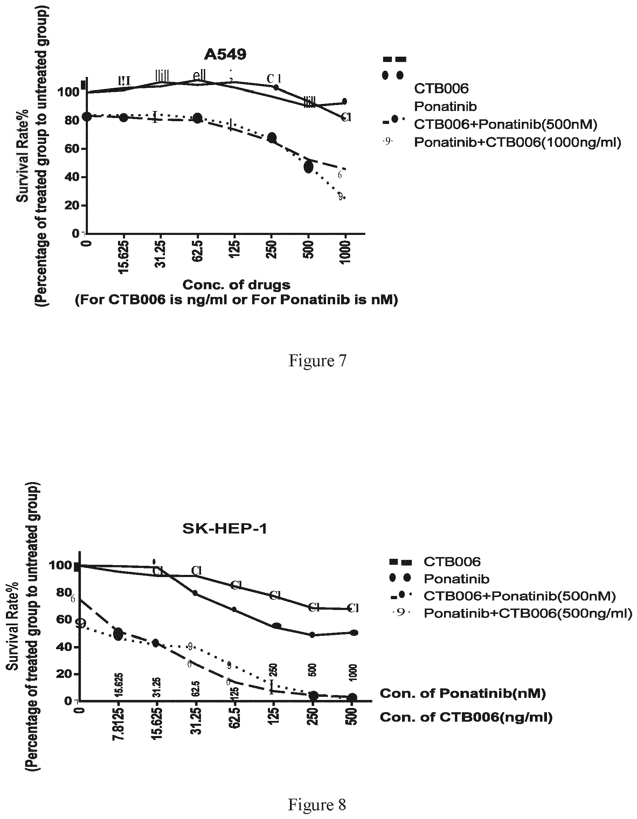

CDI values were less than 1 for co-administration of different concentrations of CTB006 antibody and 500 nM of Ponatinib, and less than 1 for co-administration of different concentrations of Ponatinib and 1000 ng/ml of CTB006 (see Table 7), indicating that the effect of co-administration of CTB006 antibody and Ponatinib on lung cancer A549 cells was synergistic (see Figure 7).

**Table 7: Survival rate and CDI of two-drug effect of CTB006 antibody and Ponatinib on A549 cells**

| CTB006 alone | | Ponatinib alone | | Varying concentrations of CTB006+500nM Ponatinib | | | Varying concentrations of Ponatinib+1000ng/ml CTB006 | | |
|---|---|---|---|---|---|---|---|---|---|
| Con. (ng/ml) | Survival Rate (%) | Con. (nM) | Survival Rate (%) | Con. (ng/ml) | Survival Rate (%) | **CDT** | Con. (nM) | Survival Rate (%) | **CDT** |
| 0 | 100.00% | 0 | 100.00% | 0 | 83.39% | | 0 | 83.81% | |
| 15.625 | 102.98% | 15.625 | 101.54% | 15.625 | 82.45% | **0.96*** | 15.62 5 | 83.64% | **0.98*** |
| 31.25 | 104.08% | 31.25 | 106.99% | 31.25 | 80.66% | **0.93*** | 31.25 | 84.13% | **0.94*** |
| 62.5 | 108.48% | 62.5 | 105.05% | 62.5 | 80.28% | **0.89*** | 62.5 | 81.92% | **0.93*** |
| 125 | 103.28% | 125 | 107.07% | 125 | 73.66% | **0.86*** | 125 | 77.07% | **0.86*** |
| 250 | 97.10% | 250 | 104.14% | 250 | 65.36% | **0.81*** | 250 | 67.06% | **0.77*** |
| 500 | 90.20% | 500 | 93.76% | 500 | 52.31% | **0.70**** | 500 | 47.94% | **0.61**** |
| 1000 | 92.31% | 1000 | 81.32% | 1000 | 45.51% | **0.59**** | 1000 | 24.73% | **0.36**** |

| | | | | | | | | | |
|---|---|---|---|---|---|---|---|---|---|
| *synergistic effect (CDI<1); ^{∗∗}significant synergistical effect (CDI<0.7) | | | | | | | | | |

### 1.1.8 CTB006 in combination with Ponatinib could synergistically kill liver cancer cells SK-HEP-1.

CDI values were less than 1 for co-administration of different concentrations of CTB006 antibody and 500 nM of Ponatinib, and less than 1 for co-administration of different concentrations of Ponatinib and 500 ng/ml of CTB006 (see Table 8), indicating that the effect of co-administration of of CTB006 antibody and Ponatinib on liver cancer SK-HEP- 1 cells was synergistic (see Figure 8).

**Table 8: Survival rate and CDI of two-drug effect of CTB006 antibody and Ponatinib on SK-HEP-1 cells**

| CTB006 alone | | Ponatinib alone | | Varying concentrations of CTB006+500nM Ponatinib | | | Varying concentrations of Ponatinib+1000ng/ml CTB006 | | |
|---|---|---|---|---|---|---|---|---|---|
| Con. (ng/ml) | Survival Rate (%) | Con. (nM) | Survival Rate (%) | Con. (ng/ml) | Survival Rate (%) | **CDT** | Con. (nM) | Survival Rate (%) | **CDT** |
| 0 | 100.00% | 0 | 100.00% | 0 | 75.05% | | 0 | 55.32% | |
| 7.8125 | 99.59% | 15.625 | 95.48% | 7.8125 | 51.30% | **0.69**** | 15.625 | 46.42% | **0.88*** |
| 15.625 | 98.75% | 31.25 | 92.47% | 15.625 | 42.76% | **0.58**** | 31.25 | 41.60% | **0.81*** |
| 31.25 | 78.79% | 62.5 | 92.34% | 31.25 | 27.16% | **0.46**** | 62.5 | 39.75% | **0.78*** |
| 62.5 | 66.78% | 125 | 84.53% | 62.5 | 13.99% | **0.28**** | 125 | 25.72% | **0.55**** |
| 125 | 54.62% | 250 | 77.45% | 125 | 7.52% | **0.18**** | 250 | 11.84% | **0.28**** |
| 250 | 48.52% | 500 | 68.54% | 250 | 4.65% | **0.13**** | 500 | 5.72% | **0.15**** |
| 500 | 50.50% | 1000 | 67.85% | 500 | 3.38% | **0.09**** | 1000 | 1.63% | **0.04**** |

| | | | | | | | | | |
|---|---|---|---|---|---|---|---|---|---|
| *synergistic effect (CDI<1); **significant synergistical effect (CDI<0.7) | | | | | | | | | |

### 1.1.9 CTB006 in combination with Ponatinib could synergistically kill liver cancer cells Huh-1.

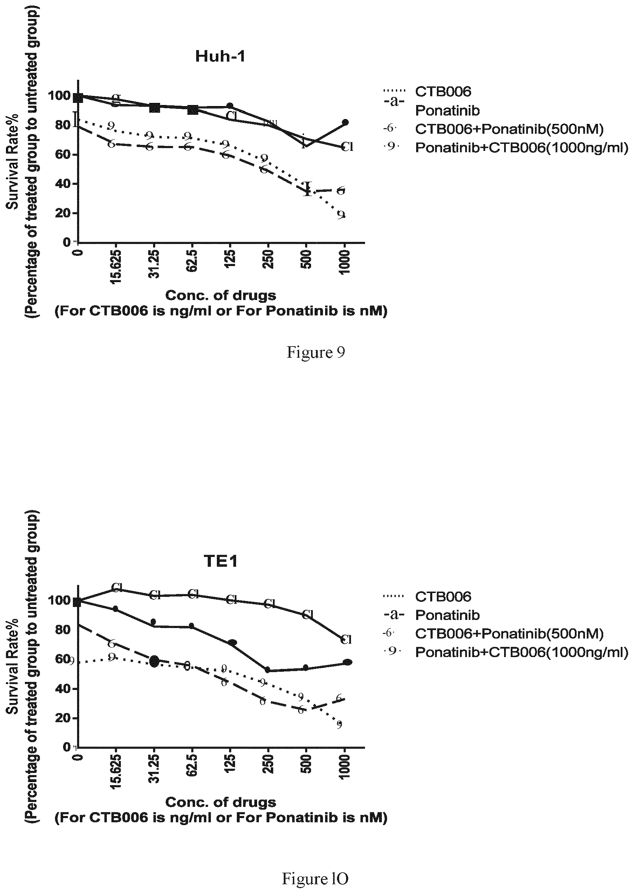

CDI values were less than 1 for co-administration of different concentrations of CTB006 antibody and 500 nM of Ponatinib and less than 1 for co-administration of different concentrations of Ponatinib and 1000 ng/ml of CTB006 (see Table 9), indicating that the effect of co-administration of CTB006 antibody and Ponatinib on liver cancer Huh-1 cells was synergistic (see Figure 9).

**Table 9: Survival rate and CDI of two-drug effect of CTB006 antibody and Ponatinib on Huh-1 cells**

| CTB006 alone | | Ponatinib alone | | Varying concentrations of CTB006+500nM Ponatinib | | | Varying concentrations of Ponatinib+1 000ng/ml CTB006 | | |
|---|---|---|---|---|---|---|---|---|---|
| Con. (ng/ml) | Survival Rate (%) | Con. (nM) | Survival Rate (%) | Con. (ng/ml) | Survival Rate (%) | **CDT** | Con. (nM) | Survival Rate (%) | **CDT** |
| 0 | 100.00% | 0 | 100.00% | 0 | 79.18% | | 0 | 83.91% | |
| 15.625 | 93.57% | 15.625 | 97.49% | 15.625 | 67.01% | **0.90*** | 15.625 | 76.06% | **0.93*** |
| 31.25 | 93.10% | 31.25 | 92.59% | 31.25 | 65.23% | **0.88*** | 31.25 | 72.05% | **0.93*** |
| 62.5 | 91.88% | 62.5 | 91.15% | 62.5 | 65.11% | **0.89*** | 62.5 | 71.25% | **0.93*** |
| 125 | 92.10% | 125 | 83.68% | 125 | 59.18% | **0.81*** | 125 | 66.18% | **0.94*** |
| 250 | 82.42% | 250 | 79.87% | 250 | 48.79% | **0.75*** | 250 | 54.54% | **0.81*** |
| 500 | 65.70% | 500 | 70.61% | 500 | 34.76% | **0.67**** | 500 | 38.61% | **0.65**** |
| 1000 | 80.63% | 1000 | 64.68% | 1000 | 36.11% | **0.57**** | 1000 | 17.31% | **0.32**** |

| | | | | | | | | | |
|---|---|---|---|---|---|---|---|---|---|
| *synergistic effect (CDI<1); **significant synergistical effect (CDI<0.7) | | | | | | | | | |

### 1.1.10 CTB006 in combination with Ponatinib could synergistically kill esophageal cancer cells TE1.

CDI values were less than 1 for co-administration of different concentrations of CTB006 antibody and 500 nM of Ponatinib and less than 1 for co-administration of different concentrations of Ponatinib and 1000 ng/ml of CTB006 (see Table 10), indicating that the effect of co-administration of CTB006 antibody and Ponatinib on esophageal cancer TE1 cells was synergistic (see Figure 10).

**Table 10: Survival rate and CDI of two-drug effect of CTB006 antibody and Ponatinib on TE1 cells**

| CTB006 alone | | Ponatinib alone | | Varying concentrations of CTB006+500nM Ponatinib | | | Varying concentrations of Ponatinib+1000ng/ml CTB006 | | |
|---|---|---|---|---|---|---|---|---|---|
| Con. (ng/ml) | Survival Rate (%) | Con. Survival (nM) Rate (%) | | Con. (ng/ml) | Survival Rate (%) | **CDT** | Con. (nM) | Survival Rate (%) | **CDT** |
| 0 | 100.00% | 0 | 100.00% | 0 | 83.65% | | 0 | 57.75% | |
| 15.625 | 93.67% | 15.625 | 107.94% | 15.625 | 70.29% | **0.90*** | 15.625 | 60.71% | **0.97*** |
| 31.25 | 82.32% | 31.25 | 103.25% | 31.25 | 59.64% | **0.87*** | 31.25 | 56.43% | **0.95*** |
| 62.5 | 81.92% | 62.5 | 104.04% | 62.5 | 55.57% | **0.81*** | 62.5 | 54.34% | **0.90*** |
| 125 | 70.32% | 125 | 100.27% | 125 | 44.33% | **0.75*** | 125 | 52.06% | **0.90*** |
| 250 | 52.09% | 250 | 97.30% | 250 | 31.26% | **0.72*** | 250 | 43.28% | **0.77*** |
| 500 | 53.50% | 500 | 89.97% | 500 | 25.75% | **0.58**** | 500 | 32.66% | **0.63**** |
| 1000 | 57.07% | 1000 | 73.00% | 1000 | 33.12% | **0.69**** | 1000 | 14.60% | **0.35**** |

| | | | | | | | | | |
|---|---|---|---|---|---|---|---|---|---|
| *synergistic effect (CDI<1); **significant synergistical effect (CDI<0.7) | | | | | | | | | |

### 10.2CTB006 in combination with Ponatinib significantly enhanced the killing effect on primary tumor cells from ovarian cancer ascites.

The tumor cells from the ascites of ovarian cancer patients were enriched and the growth of tumor cells was observed under different drug treatments.

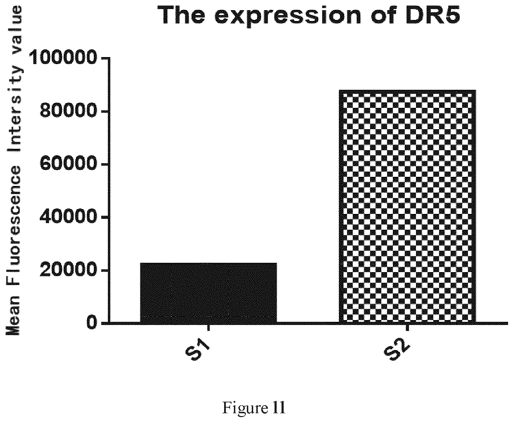

Figure 11 showed DR5 expression of primary tumor cells S1 and S2 isolated from the ascites of two ovarian cancer patients. It was found by flow cytometric detection that both S1 and S2 expressed DR5, but the abundance was different, and the expression of S2 was four times that of S1.

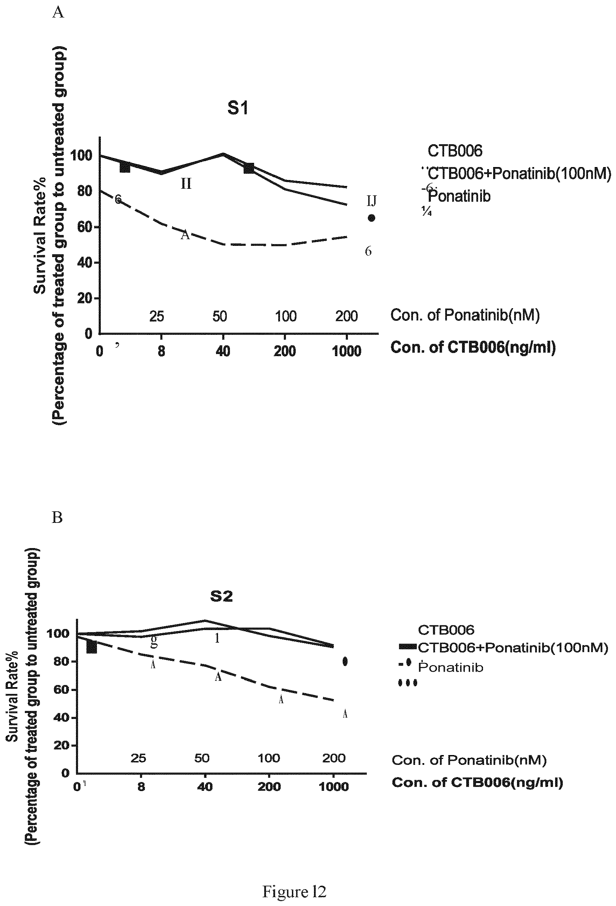

As shown in Figures 12A and 12B, CTB006 and Ponatinib had limited killing effect on both S1 and S2 when used alone. Co-administration of different concentrations of CTB006 antibody with 100 nM of Ponatinib resulted in a significant reduction of tumor cells with CDI values less than 1 (see Tables 11 and 12), indicating that co-administration of CTB006 antibody and Ponatinib synergistically enhanced the killing effect on primary tumor cells from ovarian cancer ascites.

**Table 11: Survival rate and CDI of two-drug effect of CTB006 antibody and Ponatinib on primary tumor cells S1**

| CTB006 alone | | Ponatinib alone | | Varying concentrations of CTB006+500nM Ponatinib | | |
|---|---|---|---|---|---|---|
| Con. Of CTB006(ng/ml) | Survival Rate (%) | Con. Of Ponatinib(nM) | Survival Rate (%) | Con. Of CTB006(ng/ml) | Survival Rate (%) | **CDT** |
| 0 | 100.00% | 0 | 100.00% | 0 | 80.32% | |
| 8 | 90.89% | 25 | 89.60% | 8 | 61.63% | **0.84*** |
| 40 | 100.26% | 50 | 101.09% | 40 | 50.24% | **0.62**** |
| 200 | 81.02% | 100 | 85.81% | 200 | 49.82% | **0.77*** |
| 1000 | 72.37% | 200 | 82.23% | 1000 | 54.54% | **0.94*** |

| | | | | | | |
|---|---|---|---|---|---|---|
| *synergistic effect (CDI<1); **significant synergistical effect (CDI<0.7) | | | | | | |

**Table 12: Survival rate and CDI of two-drug effect of CTB006 antibody and Ponatinib on primary tumor cells S2**

| CTB006 alone | | Ponatinib alone | | Varying concentrations of CTB006+500nM Ponatinib | | |
|---|---|---|---|---|---|---|
| Con. Of CTB006(ng/ml) | Survival Rate (%) | Con. Of Ponatinib(nM) | Survival Rate (%) | Con. Of CTB006(ng/ml) | Survival Rate (%) | **CDT** |
| 0 | 100.00% | 0 | 100.00% | 0 | 97.89% | |
| 8 | 97.86% | 25 | 101.93% | 8 | 85.23% | **0.89*** |
| 40 | 103.67% | 50 | 109.27% | 40 | 77.35% | **0.76*** |
| 200 | 103.76% | 100 | 98.46% | 200 | 62.25% | **0.61**** |
| 1000 | 91.70% | 200 | 90.46% | 1000 | 52.76% | **0.59**** |

| | | | | | | |
|---|---|---|---|---|---|---|
| *synergistic effect (CDI<1); ^{∗∗}significant synergistical effect (CDI<0.7) | | | | | | |

### Example 2 In vivo pharmacodynamic experimental data of CTB006 in combination with Ponatinib

To test the in vivo pharmacodynamic efficacy of CTB006 in combination with Ponatinib, several tumor animal models were selected for testing, including pancreatic cancer cells MIA-PACA-2 CDX model and multiple PDX models covering multiple cancers of intestinal cancer (CS237, CS263, CS264), lung cancer (CS225), and gastric cancer (CS266, GAS033). For mathematical statistics, ANOVA analysis was used, and P<0.05 indicates a statistically significant difference and is marked with ^{∗}; P<0.01 indicates a highly significant statistical difference and is marked with ^{∗∗}.

### 2.1 CTB006 in combination with Ponatinib significantly prolonged tumor growth after drug withdrawal in a pancreatic cancer CDX model.

The CDX model was established by subcutaneous transplantation of MIA-Paca-2 in BALB/c nude mice (Vital River), and drug administration was started when the tumor volume was about 0.1 cm³, and when it reached 1 cm³, the animals were ethically euthanized and survival time was recorded. CTB006 (10 mg/kg, iv, qw) and Ponatinib (30 mg/kg, p.o, qd) were administered alone or in combination for treatment groups, and the control group was administered an equal volume of saline. In this experiment, Administrations were stopped after continuous administration for 22 days, and the control group was euthanized, while the remaining groups were continuously observed until the corresponding endpoint was reached.

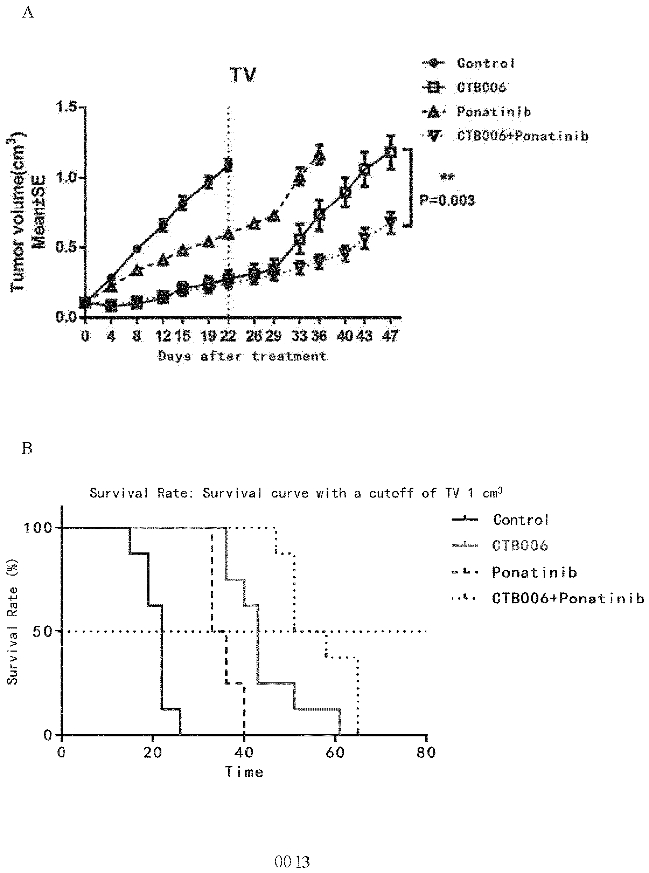

The results were shown in Figure 13A. During the administration, CTB006 alone and the combination group both showed good inhibition of tumor growth, with no significant difference between them. After withdrawal of the drug, the combination group of CTB006 and Ponatinib had more effective inhibition of tumor growth than CTB006 alone (Independent T-test, p=0.003, Day 47). Figure 13B shows the survival curves of the animals in each group, with a median survival time of 22 days in the control group and 43, 34.5 and 54.5 days in CTB006, Ponatinib and the combination group of CTB006 and Ponatinib, respectively. The combination showed a significant survival benefit.

These results suggested that the combination of CTB006 with Ponatinib significantly prolonged the growth of CDX tumor models after drug withdrawal, showing a more effective treatment effect. CTB006 may potentially enhance the efficacy in patients who are intolerant to Ponatinib.

### 2.2 Combination of CTB006 and Ponatinib significantly inhibited the growth of PDX tumor transplantation tumor model.

Patient-derived transplantation tumor models were established subcutaneously in nude mice or NOD/SCID mice, which was administered with CTB006 alone (10 mg/kg, iv, qd), Ponatinib alone (30 mg/kg, p.o, qd) the combination of them, while the control group was given an equal volume of saline. The tumor growth of the animals in each group was observed and the drug efficacy was evaluated.

The results showed that the combination of CTB006 and Ponatinib significantly inhibited tumor growth in several PDX models compared with the drug alone, and this effect persisted after drug withdrawal, showing an additive or even synergistic effect between the two drugs.

### 2.2.1 Efficacy on colorectal cancer PDX model CS237

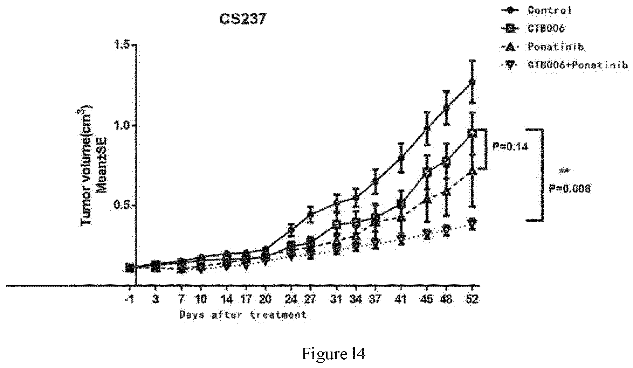

The results were shown in Figure 14. At day 52 after administration, the tumor inhibition rate (TGI) was 25.16%, 43.8%, and 70% in CTB006 alone group, Ponatinib alone group, and CTB006 and Ponatinib combination treatment group, respectively. It indicated that the combination of CTB006 and Ponatinib had better therapeutic effect.

### 2.2.2 Effects on colorectal cancer PDX model CS263

In the rectal cancer PDX model CS263, at day 44 after administration, the negative control group had a mean tumor volume of 1.5 cm³ and was ethically euthanized, and the administration was stopped for each of the remaining treatment groups and the follow-up effect continued to be observed.

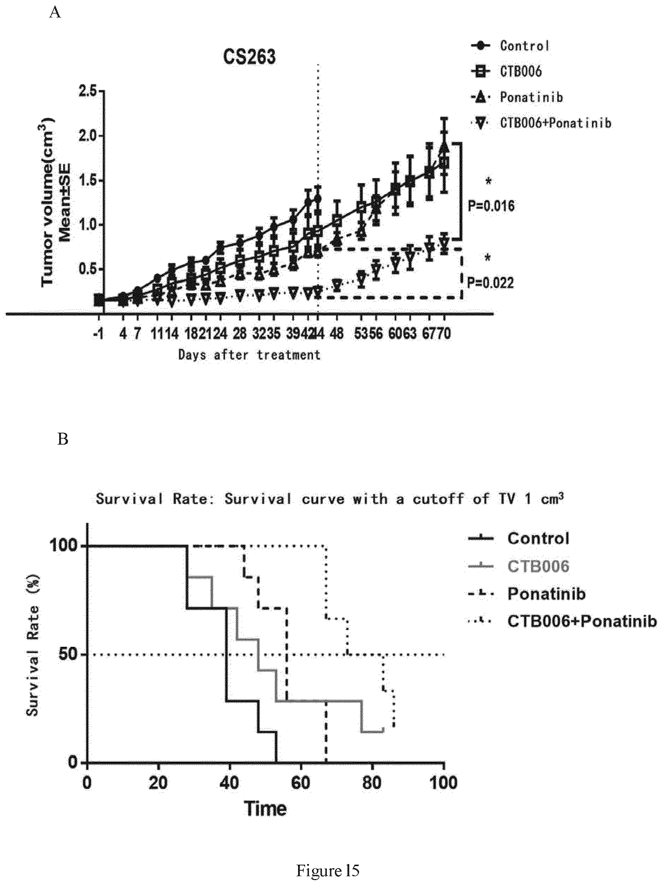

At day 44 after administration, the tumor inhibition rates (TGI) were 27.8%, 45.3%, and 80.8% in the G2 group: CTB006 alone, G3 group: Ponatinib alone, and G4 group: the combination of CTB006 and Ponatinib, respectively, and the combination of CTB006 and Ponatinib showed better therapeutic effects than the drug alone group (Figure 15A) (G2 vs G4, P=0.05; G3 vs G4, P=0.02; ANOVADunettT3).

During the continued observation after drug withdrawal, the tumor growth was found to be significantly accelerated in the Ponatinib alone group, while the tumor growth in the combination group was slightly accelerated and was more moderate compared to the drug alone group. Survival curves were made using the time to reach the mean tumor volume of 1.0 cm³ as cutoff in each group, and the median survival was 39 days in the control group, 48 days in CTB006 alone group, 56 days in Ponatinib alone group, and 78 days in CTB006 and Ponatinib combination group (Figure 15B).

The above results suggested that CTB006 with Ponatinib combination group had better results in treating the PDX transplantation tumor model CS263.

### 2.2.3 Effects on colorectal cancer PDX model CS264

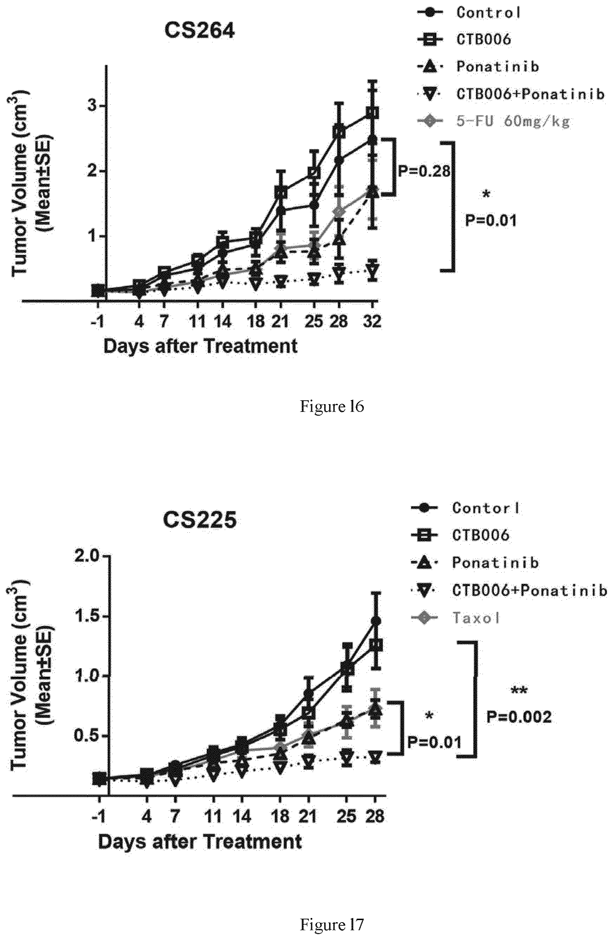

The results were shown in Figure 16. At day 32 after administration, the tumor inhibition rates (TGI) were 31.2%, -16%, 32.4%, and 80.9% in 5-Fu positive control group, CTB006 alone group, Ponatinib alone group and CTB006 and Ponatinib combination group, respectively. CTB006 alone did not show a therapeutic effect in this model, but its combination with Ponatinib significantly enhanced the therapeutic effect of Ponatinib.

These results suggested that CTB006 and Ponatinib combination group had better therapeutic effect in treating the PDX transplantation tumor model CS264.

### 2.2.4 Effects on lung cancer PDX model CS225

The results were shown in Figure 17. At day 28 after administration, the tumor inhibition rates (TGI) were 50%, 14%, 50%, 32.4%, and 77% in Taxol positive control group, CTB006 alone group, Ponatinib alone group and CTB006 and Ponatinib combination group, respectively.

These results suggested that CTB006 with Ponatinib combination group had better results in treating the PDX transplantation tumor model CS225.

### 2.2.5 Effects on gastric cancer PDX model CS266

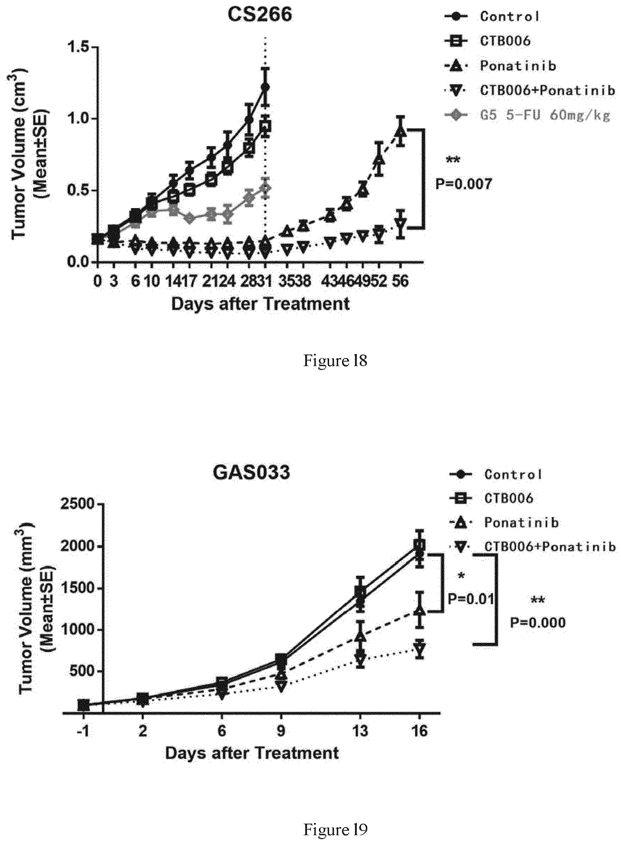

The results were shown in Figure 18. At day 31 after administration, when the mean tumor volume of the negative control group and CTB006 alone group reached 1 cm³, the negative control group was sacrificed, and the administration was stopped for 5-Fu positive control group, Ponatinib alone group and the combination group and the observation continued.

At day 31, the tumor inhibition rates (TGI) in 5-Fu-positive control group, CTB006 alone group, and CTB006 and Ponatinib combination group were 57.5%, 22%, 87.7%, and 94.6%, respectively.

During the continued observation after drug withdrawal, it was found that the tumor growth in Ponatinib alone group was significantly accelerated and the tumor suppressive effect was quickly lost, while the tumor growth rate in the combination group did not change significantly and the tumor growth inhibition was maintained.

The above results suggested that CTB006 and Ponatinib combination group had better effect in treating the PDX transplantation tumor model CS266.

### 2.2.6 Effects on gastric cancer PDX model GAS033

The results were shown in Figure 19. At day 16 after administration, the tumor inhibition rate (TGI) in CTB006 alone group, Ponatinib alone group, CTB006 and Ponatinib combination group were -3.0%, 37%, 61%, respectively.

The above results indicated that CTB006 and Ponatinib combination group had better effect in treating the PDX transplantation tumor model GAS033.

### Example 3. Co-administration of Ponatinib and other DR5 agonists

To verify whether the synergistic effect of Ponatinib and CTB006 is applicable to all DR5 agonists, other DR5 agonists of relevant types were expressed and purified according to literatures and relevant patents, including: DR5 natural ligand TRAIL (Sino Biological); DR5 agonistic monoclonal antibody B273 (Daiichi Sankyo), Conatumumab (AMG655, Amgen); a second generation multimeric DR5 agonistic antibody Hexabody-DR5/DR5 (Hexabody^{®}, Genmab, GEN1029); DR5 bispecific antibody: DR5/DR4 (CTB003, Sinotau, see patent application PCT/CN07/001453). The effect of the combination of different types of DR5 agonists and Ponatinib was verified in vitro with multiple cells. Methods were as described above.

### 30.1 Different types of DR5 agonists in combination with Ponatinib could synergistically kill the lung cancer cells A549.

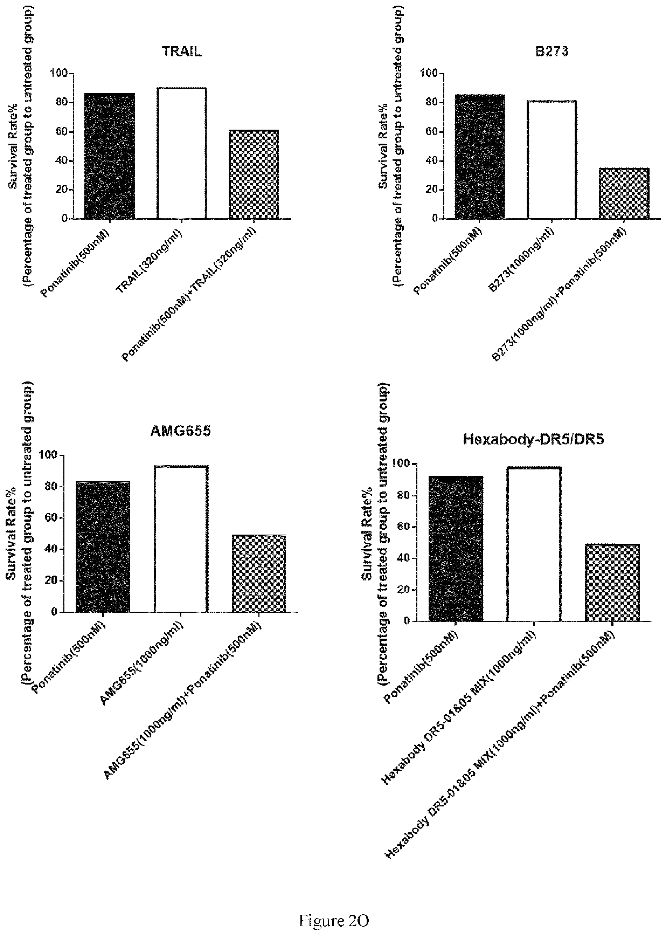

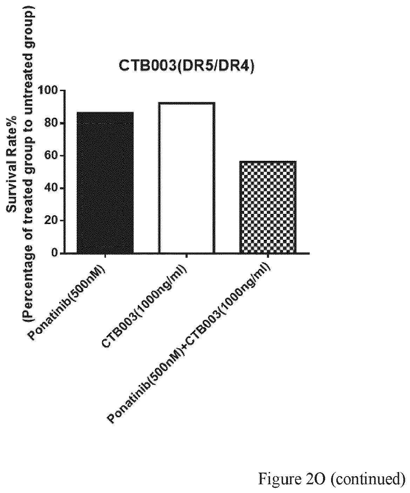

In the experiments of the lung cancer cells A549, different types of DR5 agonists and Ponatinib alone at corresponding concentrations had no killing effect on A549. When they were used in combination at the same doses, there was a significant killing effect. Their CDI values were all less than 1 (see Table 13), indicating that the effect of different types of DR5 agonists in combination with Ponatinib on lung cancer A549 cells was synergistic (see Figure 20).

**Table 13 CDI of two-drug effect of different types of DR5 agonists and Ponatinib on A549 cells**

| A549 | Ponatinib | | | | |
|---|---|---|---|---|---|
| | TRAIL | B273 | AMG655 | Hexabody-DR5/DR5 | DR5/DR4 |
| CDI | 0.732944 | 0.498266 | 0.636172 | 0.520906 | 0.561583 |

### 3.2 Different types of DR5 agonists in combination with Ponatinib could synergistically kill pancreatic cancer cells S2VP10.

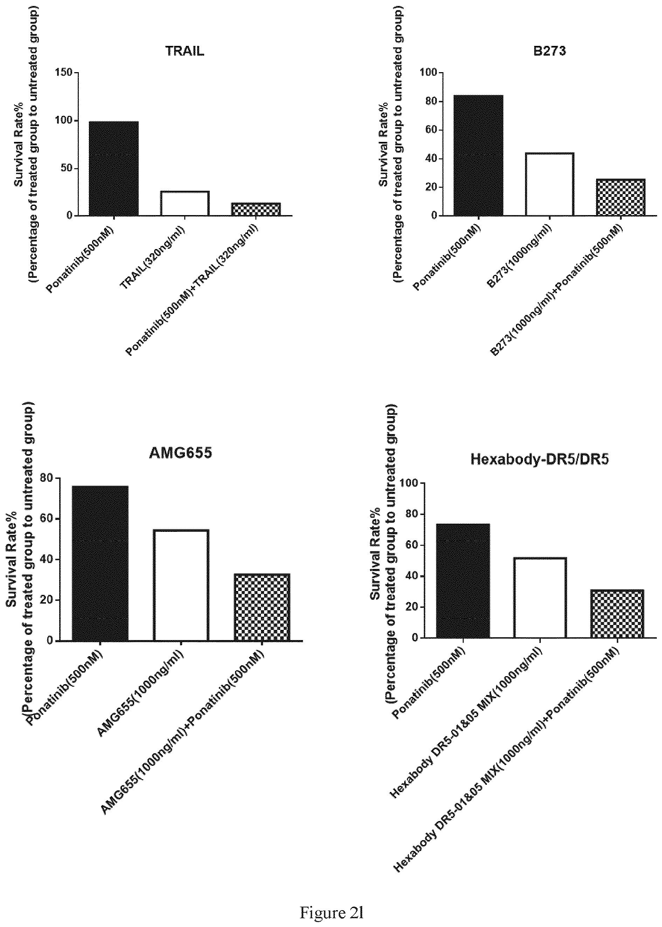

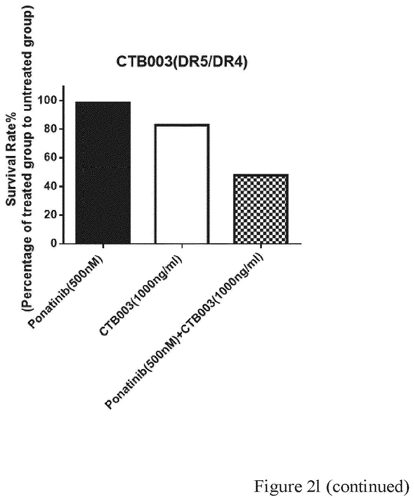

In the experiments of the pancreatic cancer cells S2VP10, the killing effect of different types of DR5 agonists and Ponatinib alone at corresponding concentrations was not significant for S2VP10. When they were used in combination at the same doses, there was a significant killing effect. Their CDI values were all less than 1 (see Table 14), indicating that the effect of different types of DR5 agonists in combination with Ponatinib on pancreatic cancer S2VP10 cells was synergistic (see Figure 21).

**Table 14 CDI of two-drug effect of different types of DR5 agonists and Ponatinib on S2VP10 cells**

| S2VP10 | Ponatinib | | | | |
|---|---|---|---|---|---|
| | TRAIL | B273 | AMG655 | Hexabody-DR5/DR5 | DR5/DR4 |
| CDI | 0.51791 | 0.692017 | 0.81231 | 0.831459 | 0.544903 |

### 3.3 Different types of DR5 agonists in combination with Ponatinib could synergistically kill breast cancer cells MCF7.

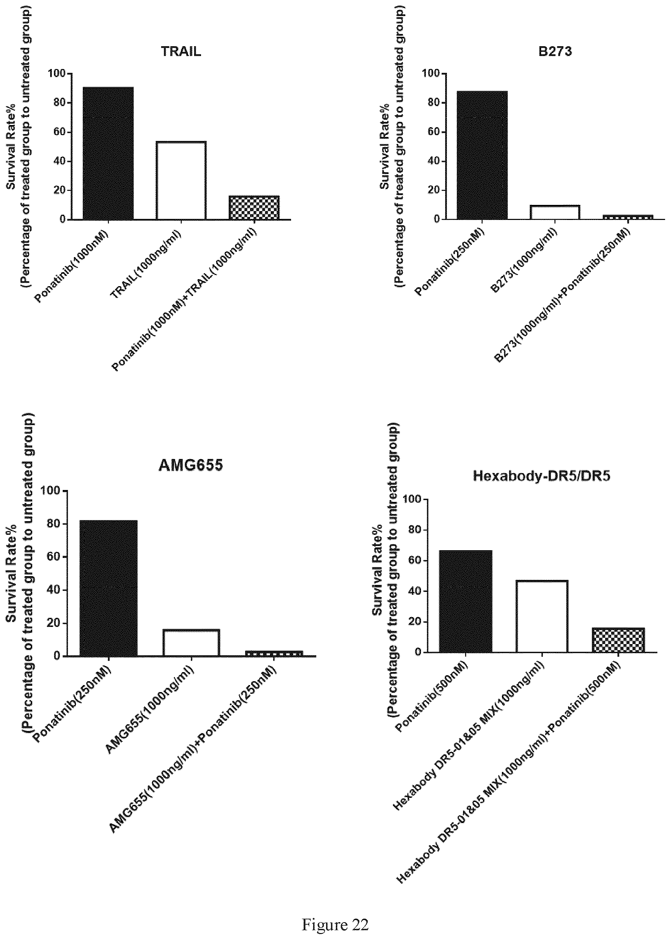

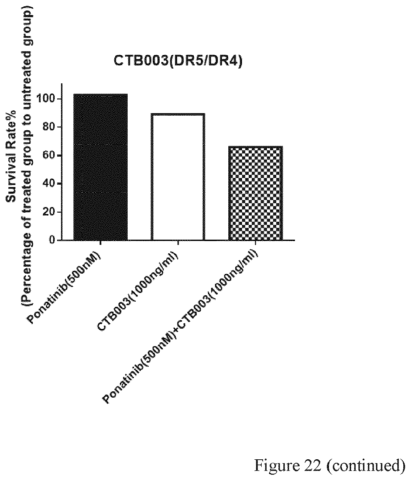

In the experiments of breast cancer cells MCF7, most types of DR5 agonists (e.g., TRAIL, Hexabody-DR5/DR5 and DR5/DR4) and Ponatinib alone at corresponding concentrations did not have a significant killing effect on MCF7. A significant killing effect was observed when they were used in combination at the same doses. In addition, B273 and AMG655 had a significant killing effect on MCF7 when used alone, while Ponatinib was ineffective when used alone, and the killing effect was more significant when the two were used in combination. Their CDI values were all less than 1 (see Table 15), indicating that the effect of different types of DR5 agonists in combination with Ponatinib on breast cancer MCF7 cells was synergistic (see Figure 22).

**Table 15 CDI of two-drug effect of different types of DR5 agonists and Ponatinib on MCF7 cells**

| MCF7 | Ponatinib | | | | |
|---|---|---|---|---|---|
| | TRAIL | B273 | AMG655 | Hexabody-DR5/DR5 | DR5/DR4 |
| CDI | 0.330277 | 0.197514 | 0.316996 | 0.68057 | 0.721373 |

### Example 4. Possible mechanism of Ponatinib in combination with CTB006

To investigate the molecular mechanism of the synergistic effect between Ponatinib and CTB006 and to investigate whether the combination of them can activate the apoptotic pathway, four tumor cells, including colon cancer cells SW1116 (A), gastric cancer cells 7901 (B), colorectal cancer HT29 (C), pancreatic cancer S2Lm7AA (D), were treated with the corresponding drugs overnight, and cell lysates were taken for Western blot, Caspase cleavage was detected, and the activation of apoptotic signaling pathway and the phosphorylation level of MEK/ERK signaling pathway were measured to reflect the cell proliferation.

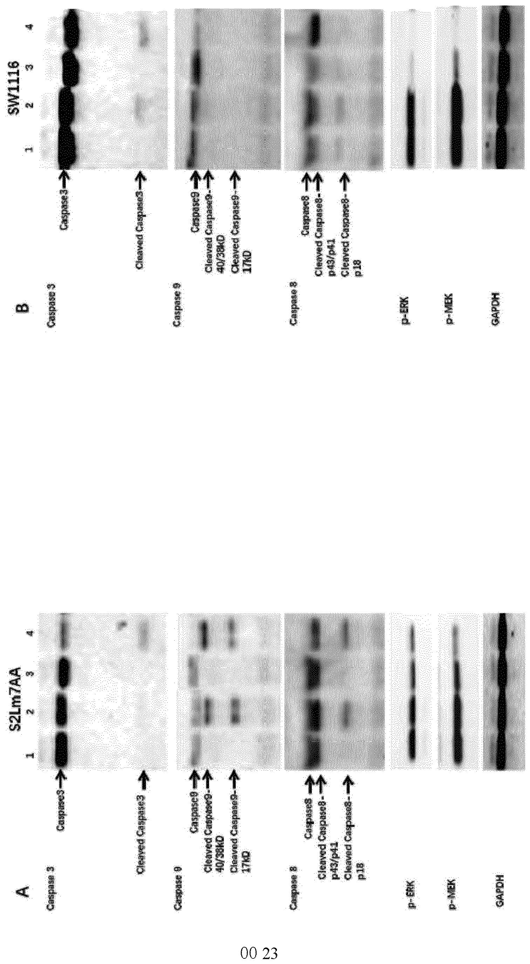

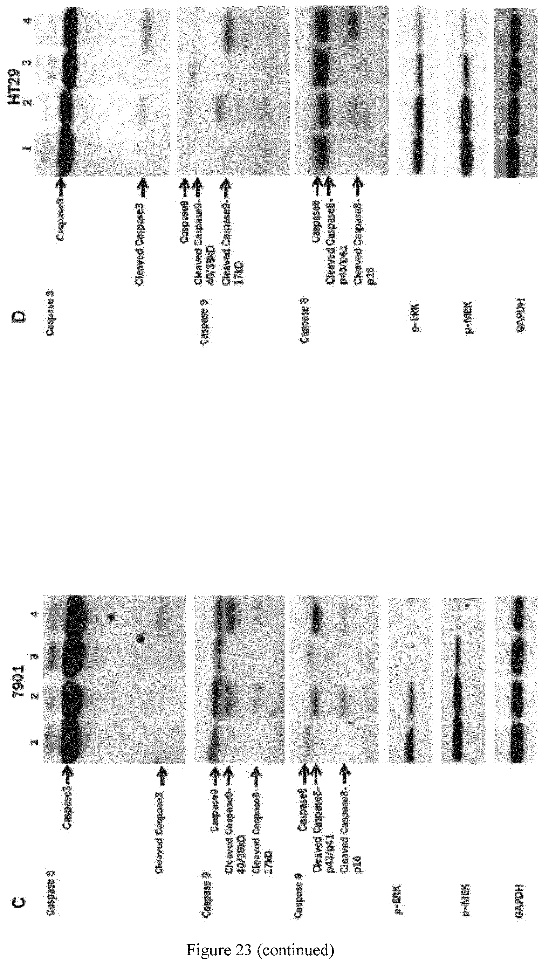

Lane 1-4 in Figure 23 were control, CTB006 treatment, Ponatinib treatment, and CTB006+Ponatinib combination treatment, respectively. Caspase-3 blot showed that in all four cells, Ponatinib alone did not induce caspase cleavage, and CTB006 either alone or in combination with Ponatinib showed significant cleavage, indicating that the cells were in an apoptotic state. Caspase-3 cleavage was enhanced by the combination of CTB006+Ponatinib than by CTB006 alone.

In 7901 (B), HT29 (C), and S2Lm7AA (D) cells, the results of caspase-8,9 cleavage were similar. Treatment with Ponatinib alone did not induce cleavage of caspase-8,9, and CTB006, either alone or in combination with Ponatinib, induced significant cleavage, suggesting that the endogenous and exogenous apoptotic pathway in the cells were both activated. In two cell lines, 7901 and S2Lm7AA, the cleavage of caspase-9, a marker of endogenous apoptosis, was also slightly enhanced by the combination of CTB006+Ponatinib than by CTB006 alone.

In the MEK/ERK pathway, in four cells, p-MEK, p-ERK were not significantly changed in the negative control group and CTB006 alone group, p-MEK, p-ERK were significantly downregulated in Ponatinib alone group, while p-MEK and p-ERK decreased more significantly in the combination group.

In conclusion, the combination of CTB006 and Ponatinib could activate Caspase signaling pathway, promote the cleavage of caspase, and activate the endogenous and exogenous apoptotic pathway of tumor cells; meanwhile, the combination could enhance the inhibition of the phosphorylation of MEK and ERK, inhibit the activation of MEK/ERK signaling pathway and thus inhibit tumor cell growth.

References
1. TANG Xiu-hong, QIN SHu-kui, CHEN Hui-ying, et al., Increasing anti-tumor effect of arsenic trioxide combining with cisplatin on the QGY-7701 human hepatocarcinoma cells line, Cancer Research on Prevention and Treatment, 2002, Vol. 29, Issue 5, 362-364.
2. Zhiyong LIAO, Shenghua ZHANG, Yongsu ZHEN, Geldanamycin enhances antitumor efficacy of cisplatin, Chinese Journal of Cancer, 2000, Vol. 19, No. 8, 731-734.

## Claims

1. A combination of antitumor medicaments comprising an apoptosis inducer and a multi-targeted kinase inhibitor.

2. The combination of antitumor medicaments according to claim 1, wherein the apoptosis inducer is a TRAIL-R2 binding agent.

3. The combination of antitumor medicaments according to claim 2, wherein the TRAIL-R2 binding agent is a TRAIL-R2 agonist.

4. The combination of antitumor medicaments according to claim 3, wherein the TRAIL-R2 agonist is a TRAIL-R2 natural ligand TRAIL or a TRAIL-R2 agonistic antibody.

5. The combination of antitumor medicaments according to claim 4, wherein the TRAIL-R2 agonistic antibody is B273, Conatumumab, CTB006 antibody, Hexabody-DR5/DR5 or CTB003 antibody.

6. The combination of antitumor medicaments according to any one of the preceding claims, wherein the multi-targeted kinase inhibitor is Ponatinib or a pharmaceutically acceptable salt thereof.

7. The combination of antitumor medicaments according to any one of the preceding claims, wherein the multi-targeted kinase inhibitor is Ponatinib hydrochloride.

8. The combination of antitumor medicaments according to any one of the preceding claims, wherein the tumor expresses TRAIL-R2 on the cell surface.

9. The combination of antitumor medicaments according to any one of the preceding claims, wherein the tumor is breast cancer, pancreatic cancer, colorectal cancer, gastric cancer, lung cancer, liver cancer, esophageal cancer or ovarian cancer.

10. The combination of antitumor medicaments according to any one of the preceding claims, wherein the apoptosis inducer and the multi-targeted kinase inhibitor are present in the same or different pharmaceutical composition or pharmaceutical kit.

11. The combination of antitumor medicaments according to any one of the preceding claims, wherein the apoptosis inducer is in a dosage form suitable for intravenous administration and the multi-targeted kinase inhibitor is in a dosage form suitable for oral administration.

12. A method of treating a tumor in a subject comprising administering to the subject a therapeutically effective amount of an apoptosis inducer and a multi-targeted kinase inhibitor simultaneously, sequentially or separately.

13. The method according to claim 12, wherein the apoptosis inducer is a TRAIL-R2 binding agent.

14. The method according to claim 13, wherein the TRAIL-R2 binding agent is a TRAIL-R2 agonist.

15. The method according to claim 14, wherein the TRAIL-R2 agonist is a TRAIL-R2 natural ligand TRAIL or a TRAIL-R2 agonistic antibody.

16. The method according to claim 15, wherein the TRAIL-R2 agonistic antibody is B273, Conatumumab, CTB006 antibody, Hexabody-DR5/DR5 or CTB003 antibody.

17. The method according to any one of claims 12-16, wherein the multi-targeted kinase inhibitor is Ponatinib or a pharmaceutically acceptable salt thereof.

18. The method according to any one of claims 12-17, wherein the multi-targeted kinase inhibitor is Ponatinib hydrochloride.

19. The method according to any one of claims 12-18, wherein the tumor expresses TRAIL-R2 on the cell surface.

20. The method according to any one of claims 12-19, wherein the tumor is breast cancer, pancreatic cancer, colorectal cancer, gastric cancer, lung cancer, liver cancer, esophageal cancer or ovarian cancer.

21. The method according to any one of claims 12-20, wherein the apoptosis inducer is administered by intravenous injection and the multi-targeted kinase inhibitor is administered orally.

22. Use of an apoptosis inducer in the preparation of a medicament for co-administration in combination with a multi-targeted kinase inhibitor to treat a tumor.

23. The use according to claim 22, wherein the apoptosis inducer is a TRAIL-R2 binding agent.

24. The use according to claim 23, wherein the TRAIL-R2 binding agent is a TRAIL-R2 agonist.

25. The use according to claim 24, wherein the TRAIL-R2 agonist is a TRAIL-R2 natural ligand TRAIL or a TRAIL-R2 agonistic antibody.

26. The use according to claim 25, wherein the TRAIL-R2 agonistic antibody is B273, Conatumumab, CTB006 antibody, Hexabody-DR5/DR5 or CTB003 antibody.

27. The use according to any one of claims 22-26, wherein the multi-targeted kinase inhibitor inhibits activation of the MEK/ERK signaling pathway.

28. The use according to any one of claims 22-27, wherein the multi-targeted kinase inhibitor is Ponatinib or a pharmaceutically acceptable salt thereof.

29. The use according to any one of claims 22-28, wherein the multi-targeted kinase inhibitor is Ponatinib hydrochloride.

30. The use according to any one of claims 22-29, wherein the tumor expresses TRAIL-R2 on the cell surface.

31. The use according to any one of claims 22-30, wherein the tumor is breast cancer, pancreatic cancer, colorectal cancer, gastric cancer, lung cancer, liver cancer, esophageal cancer or ovarian cancer.

32. A method of inducing apoptosis in a tumor cell comprising contacting said tumor cell with an apoptosis inducer and a multi-targeted kinase inhibitor.

33. The method according to claim 32, wherein the apoptosis inducer is CTB006 antibody, the multi-targeted kinase inhibitor is Ponatinib or hydrochloride thereof.

34. A method of enhancing antitumor effects in a subject intolerant to Ponatinib or a pharmaceutically acceptable salt thereof, comprising administering CTB006 antibody to the subj ect.
